(19) **Europäisches Patentamt / European Patent Office / Office européen des brevets**

(11) **EP 3 575 992 A1**

(12) **EUROPEAN PATENT APPLICATION**
published in accordance with Art. 153(4) EPC

(43) Date of publication:
**04.12.2019 Bulletin 2019/49**

(51) Int Cl.:
***G06F 19/00*** (2018.01)

(21) Application number: **18745052.3**

(86) International application number:
**PCT/JP2018/001160**

(22) Date of filing: **17.01.2018**

(87) International publication number:
**WO 2018/139300 (02.08.2018 Gazette 2018/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **24.01.2017 JP 2017010441**

(71) Applicant: **NEC Corporation**
**Tokyo 108-8001 (JP)**

(72) Inventors:
• **AZUMA, Tan**
**Tokyo 108-8001 (JP)**
• **ARAKI, Soichiro**
**Tokyo 108-8001 (JP)**
• **FUJIYAMA, Kenichiro**
**Tokyo 108-8001 (JP)**
• **SATOH, Mineto**
**Tokyo 108-8001 (JP)**
• **ARIYAMA, Tetsuri**
**Tokyo 108-8001 (JP)**

(74) Representative: **Vossius & Partner**
**Patentanwälte Rechtsanwälte mbB**
**Siebertstrasse 3**
**81675 München (DE)**

(54) **INFORMATION PROCESSING DEVICE, INFORMATION PROCESSING METHOD, AND RECORDING MEDIUM HAVING INFORMATION PROCESSING PROGRAM RECORDED THEREON**

(57) Provided are an information processing device and the like for providing information that serves as a basis for estimating a state relating to an object with high precision. The information processing device 401 comprises: a relationship determination unit 402 for selecting sets satisfying a first condition stipulating that the distance is not far from other sets or that said sets are similar to other sets in a distribution of at least some sets from among a plurality of sets in which state information representing a state relating to an object and the likelihood of the occurrence of a scenario representing a situation in which the state information changes are associated; and an evaluation processing unit 403 for determining the state information corresponding to the likelihood of occurrence in sets satisfying a predetermined selection condition from among the sets selected by the relationship determination means.

Fig.6

**Description**

[Technical Field]

The present invention relates to an information processing apparatus and the like of predicting, for example, an event that occurs on a target.

[Background Art]

An example of estimating a state (or an event) of a simulation target (hereinafter, represented as a "target") is a simulation method which adjusts parameters of a mathematical model representing a state of the target in such a way as to suit to observation information observed for the target, and thereby simulates the target. Alternatively, an example of one evolution systems of the above-described simulation is a simulation method of representing uncertainty for a target by using a probability distribution of parameters and acquiring data in such a way that a value close to observation information is calculated by using a model including the parameters.

In the following description, it is assumed that an event or a state is generically represented as a "state".

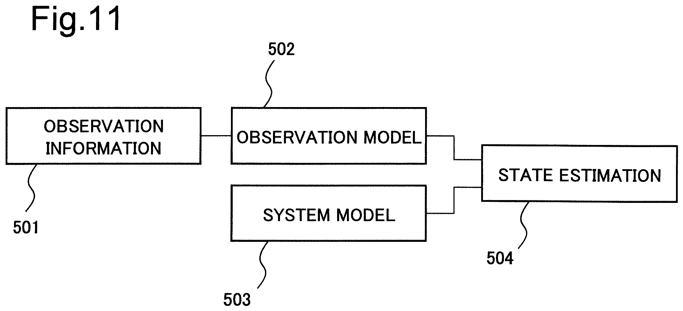

With reference to Fig. 11, the latter simulation method will be more specifically described. Fig. 11 is a diagram conceptually illustrating a process in a simulation method. The simulation method includes a process of generating information in which observation information 501 input to an observation model 502 is integrated with a system model 503 being a mathematical simulation model and a process of estimating a state of a target by using the generated information. The observation information 501 is information observed in relation to a target. The observation model 502 is a mathematical model representing a state of the target. An estimation process (state estimation 504) of a target state in the simulation method includes a process of acquiring a probability distribution suitable to the observation information (an observation value) 501 observed for the target among probability distributions relating to a variable (parameter) included in the observation model 502 or the system model 503. Then, the simulation method includes the state estimation 504 of acquiring a value of an objective variable representing a state to be a remarkable state of the target based on the acquired probability distribution. In the following description, prediction information (a prediction value) and estimation information (an estimation value) acquired for an objective variable are referred to as "analysis information".

The latter simulation method includes a process of calculating, when time series data are input, an influence of the observation information 501 on analysis information in an order of timings in the time series data This process is referred to as "filtering". An example of filter in the filtering is a particle filter, an ensemble Kalman filter, and the like.

In the following description, a probability distribution of a variable value before a filtering process is referred to as a "prior probability distribution" (or a "prior distribution"). A probability distribution of a variable value after a filtering process is referred to as a "posterior probability distribution" (or a "posterior distribution").

PTL 1 and PTL 2 disclose a simulation technology for estimating a state of a target by acquiring a likely state while using filtering. A method of acquiring a likely state is also referred to as a maximum likelihood estimation method.

In a process of acquiring a posterior probability distribution in filtering, an apparatus disclosed in PTL 1 calculates, based on an error between observation information observed for a target and a prior probability distribution for the target, a likelihood for the observation information. The apparatus generates the posterior probability distribution by applying weight depending on the calculated likelihood to the prior probability distribution.

An apparatus disclosed in PTL 2 includes a plurality of observation models for a target and a determination unit. In a data assimilation process using filtering, the determination unit selects a likely result based on a plurality of posterior distributions in the plurality of observation models. The apparatus may accurately simulate a state of a target even in case of non-ideal observation information (e.g., data including noise).

[Citation List]

[Patent Literature]

PTL 1: Japanese Patent Publication No. 5340228
PTL 2: International Publication No. WO 2016/031174

[Summary of Invention]

[Technical Problem]

However, even when the apparatus disclosed in PTL 1 or PTL 2 is used, difficulty is to perform prediction for a target accurately. In other words, even when these apparatuses are used, difficulty is to solve a problem of an error occurring between information (or a value) actually observed for target and analysis information predicted for the target. A reason for this is that, even when these apparatuses acquires a state of a target by selecting a likely state, a divergence occurs between the acquired state and an actual state occurring for the target.

Thus, one object of the present invention is to provide an information processing apparatus and the like which provide information serving as a basis for accurately estimating a state of a target.

[Solution to Problem]

As an aspect of the present invention, an information processing apparatus includes:

relevance determination means for determining relevance between status information representing a state of a target and a possibility of a scenario representing aspect of change of the state information; and
evaluation processing means for determining state information in case that the possibility satisfies a predetermined selection criterion based on the relevance determined by the relevance determination means.

In addition, as another aspect of the present invention, an information processing method includes:

determining relevance between status information representing a state of a target and a possibility of a scenario representing aspect of change of the state information; and
determining state information in case that the possibility satisfies a predetermined selection criterion based on the determined relevance.

In addition, as another aspect of the present invention, an information processing program causes a computer to achieve:

a relevance determination function for determining relevance between status information representing a state of a target and a possibility of a scenario representing aspect of change of the state information; and
an evaluation processing function for determining state information in case that the possibility satisfies a predetermined selection criterion based on the relevance determined by the relevance determination function.

Furthermore, the object is also achieved by a computer-readable recording medium that records the program.

[Advantageous effects of Invention]

An information processing apparatus and the like according to the present invention is able to provide information serving as a basis for accurately estimating a state of a target.

[Brief Description of Drawings]

[Fig. 1] Fig. 1 is a block diagram illustrating a configuration of an information processing apparatus according to a first example embodiment of the present invention.
[Fig. 2] Fig. 2 is a flowchart illustrating flow of a process in the information processing apparatus according to the first example embodiment.
[Fig. 3] Fig. 3 is a diagram conceptually illustrating a relevance acquired by a fitting process based on a state value relating to a state in a scenario and an evaluation value for the scenario.
[Fig. 4] Fig. 4 is a block diagram illustrating a configuration of an information processing apparatus according to a second example embodiment of the present invention.
[Fig. 5] Fig. 5 is a flowchart illustrating flow of a process in the information processing apparatus according to the second example embodiment.
[Fig. 6] Fig. 6 is a block diagram illustrating a configuration of an information processing apparatus according to a third example embodiment of the present invention.

[Fig. 7] Fig. 7 is a flowchart illustrating flow of a process in the information processing apparatus according to the third example embodiment.

[Fig. 8] Fig. 8 is a block diagram illustrating a configuration of an information processing apparatus according to a fourth example embodiment of the present invention.

[Fig. 9] Fig. 9 is a flowchart illustrating flow of a process in the information processing apparatus according to the fourth example embodiment.

[Fig. 10] Fig. 10 is a block diagram schematically illustrating a hardware configuration of a calculation processing apparatus capable of achieving information processing apparatus according to each example embodiment of the present invention.

[Fig. 11] Fig. 11 is a diagram conceptually illustrating a process in a simulation method.

[Example Embodiment]

In order to facilitate understanding of the invention of the present application, technical terms and a problem to be solved by the invention of the present application will be described. First, technical terms will be described. In the following description, it is assumed that an event or a state is generically referred to as a "state".

Data assimilation technology is a technology for introducing information representing uncertainty to a mathematical model representing a state of a target changing as time elapses in such a way as to match to observation information for the target and, thereby, achieving higher prediction accuracy. A model in data assimilation technology includes a plurality of parameters and a state variable. A value is set for each of the plurality of parameters. Further, a value of a state variable at each timing is set for each state variable. Hereinafter, a situation where a value of a parameter and a value (hereinafter, also referred to as a state value or state information) of a state variable change as time elapses is referred to as a "scenario". In other words, a scenario represents an aspect of state change of a target. In data assimilation technology, it is assumed that a value of a state variable is distributed following a probability distribution. As a result, a plurality of scenarios estimated by the model exist.

Hereinafter, a simulation in accordance with one scenario or a scenario itself is referred to as a "particle". For example, a plurality of particles represent multiple simulation in accordance with the plurality of respective scenarios (or scenarios themselves). Moreover, in a scenario, values of a plurality of parameters and a value of a state variable at a certain timing are referred to as a "state at the certain timing".

Simulation technology such as data assimilation technology probabilistically predicts a possible state actually occurring for a target. The simulation technology predicts, for example, that a probability that a state at the timing t is the state C is 80% when a ratio of particles for a scenario satisfying a condition that a state at a timing t is a state C is 80% of all particles. A plurality of particles for one scenario may exist.

Analysis information represents a value of a state variable acquired based on a mathematical model for a target. Observation information represents, for example, observation information (observation value) observed by an observation apparatus observing the target. A model value represents a value of a variable representing a state in one scenario in a model in which a value of a variable included in a mathematical model is distributed following a probability distribution.

For example, the simulation technology as described above includes a process of replicating or deleting a particle in such a way as to generate, in relation to a plurality of model values at a certain timing, a probability distribution in which observation information at the certain timing is generated. Alternatively, the simulation technology includes a process of changing a scenario relating to a particle in such a way as to generate, in relation to a plurality of model values at a certain timing, a probability distribution in which observation information at the certain timing is generated. Hereinafter, a process of replicating, deleting, or changing a particle is referred to as a "filtering process". An example of method for implementing the filtering process is a particle filter, an ensemble Kalman filter or the like. In these methods, when analysis information for a target is calculated, a particle most suitable (fitting, likely) to observation information at one timing is selected among a plurality of particles. Hereinafter, a fitting degree is referred to as a "likelihood". Herein, each likelihood calculated in an information processing apparatus according to each example embodiment of the present invention will be described.

A first likelihood (likelihood for each piece of observation information relating to a certain timing) represents a degree (level) at which a variable $x_{t|t-1}^{(i)}$ (i is a natural number) for an i-th particle is suitable to observation information $y_{j,t}$ observed by a j-th (j is a natural number) observation apparatus at a certain timing t (t is a natural number), as indicated by Eqn. 1.

$$\lambda_{j,t}^{(i)} = p\left(y_{j,t} \mid x_{t|t-1}^{(i)}\right) \quad \cdots \left(\text{Eqn.1}\right)$$

p(A|B) represents a conditional probability at which an event A occurs when an event B occurs. $x_{t|t-1}^{(i)}$ represents a state, for an i-th particle, estimated in relation to the timing t, based on a state at a timing (t-1) and a mathematical model.

Therefore, the first likelihood represents a degree at which analysis information is suitable to the observation information $y_{j,t}$ at the timing t in an i-th scenario.

A second likelihood (comprehensive likelihood relating to a certain timing) represents a degree (level) at which a variable $x_{t|t-1}^{(i)}$ for an i-th particle is suitable to all pieces of observation information $y_t$ at the certain timing t, as indicated by Eqn. 2.

$$\lambda_t^{(i)} = \prod_j \lambda_{j,t}^{(i)} \quad \cdots \quad (\text{Eqn.}2)$$

However, $\Pi_j$ represents multiplication relating to j.

Therefore, the second likelihood represents a degree at which analysis information is suitable to all pieces of observation information at the timing t in the i-th scenario.

A third likelihood (likelihood for each piece of observation information during a certain period) represents an infinite product of the first likelihood (Eqn. 1) in a period (t_1→t_2) from a timing t1 to a timing t2, as indicated by Eqn. 3.

$$\lambda_{j,t_1 \to t_2}^{(i)} = \prod_{t=t_1}^{t_2} \lambda_{j,t}^{(i)} \quad \cdots \quad (\text{Eqn.}3)$$

Therefore, the third likelihood represents a degree at which analysis information is suitable to the observation information $y_{j,t}$ in a period from the timing t1 to the timing t2 in the i-th scenario.

A fourth likelihood (comprehensive likelihood during a certain period) represents an infinite product of the second likelihood (Eqn. 2) in a period (t1→t2) from the timing t1 to the timing t2, as indicated by Eqn. 4.

$$\lambda_{t_1 \to t_2}^{(i)} = \prod_{t=t_1}^{t_2} \lambda_t^{(i)} \quad \cdots \quad (\text{Eqn.}4)$$

In other words, the fourth likelihood represents a degree at which analysis information is suitable to all pieces of observation information in a period from the timing t1 to the timing t2 in the i-th scenario. The third likelihood and the fourth likelihood is not necessarily calculated in a processing procedure by taking an infinite product as exemplified by Eqn. 3 and Eqn. 4, and may be calculated in, for example, a processing procedure by taking a summation. Processing procedures of calculating the third likelihood and the fourth likelihood are not limited to Eqn. 3 and Eqn. 4.

As indicated by Eqns. 5 to 8, a particle average likelihood is calculated by an average value relating to all scenarios in relation to the above-described four first to fourth likelihoods, respectively.

$$L_{j,t} = \frac{1}{N} \sum_{i=1}^{N} \lambda_{j,t}^{(i)} \quad \cdots \quad (\text{Eqn.}5)$$

$$L_t = \frac{1}{N} \sum_{i=1}^{N} \lambda_t^{(i)} \quad \cdots \quad (\text{Eqn.}6)$$

$$L_{j,t_1 \to t_2} = \frac{1}{N} \sum_{i=1}^{N} \lambda_{j,t_1 \to t_2}^{(i)} \qquad \bullet \quad \bullet \quad \bullet \,(\text{Eqn.}7)$$

$$L_{t_1 \to t_2} = \frac{1}{N} \sum_{i=1}^{N} \lambda_{t_1 \to t_2}^{(i)} \qquad \bullet \quad \bullet \quad \bullet \,(\text{Eqn.}8)$$

$\Sigma$ represents a process of calculating a summation.

A first average likelihood indicated by Eqn. 5 represents an average value of a fitting degree of analysis information to the observation information $y_{j,t}$ at the timing t on all scenarios. A second average likelihood indicated by Eqn. 6 represents an average value of a fitting degree of analysis information to all pieces of observation information at the timing t on all scenarios. A third average likelihood indicated by Eqn. 7 represents an average value of a fitting degree of analysis information to the observation information $y_{j,t}$ in a period from the timing t1 to the timing t2 on all scenarios. A fourth average likelihood indicated by Eqn. 8 represents an average value of a degree of analysis information to all pieces of observation information in a period from the timing t1 to the timing t2 on all scenarios.

Next, a problem found out by the inventor of the present application will be described.

A maximum likelihood estimation method of each of PTL 1, PTL 2, and the like includes a process of selecting a particle having a high likelihood at one timing among a plurality of particles. However, a simulation in accordance with a scenario relating to a particle having a highest likelihood at one timing does not necessarily have high prediction accuracy relating to a target. The inventor of the present application has found out that one of the causes that prediction accuracy for a target does not become high lies in a fact that uncertainty in a simulation model is represented by using a finite discrete distribution. Further, the inventor of the present application has found out that there is a statistical relevance between an evaluation value (described later with reference to Eqn. 9 and the like) such as a likelihood of a particle and analysis information, and even when a particle deviating from the relevance is a particle having a high likelihood, a state estimated in accordance with a scenario for the particle becomes a state divergent from actual observation information. Therefore, the inventor of the present application has found out a problem that even a simulation in accordance with a scenario for a particle having a high likelihood has low prediction accuracy using the simulation.

The inventor of the present application has found the problem, and arrived at deriving a means for solving the problem. Hereinafter, an example embodiment of the present invention being capable of solving such a problem will be described in detail with reference to the drawings.

<First example embodiment>

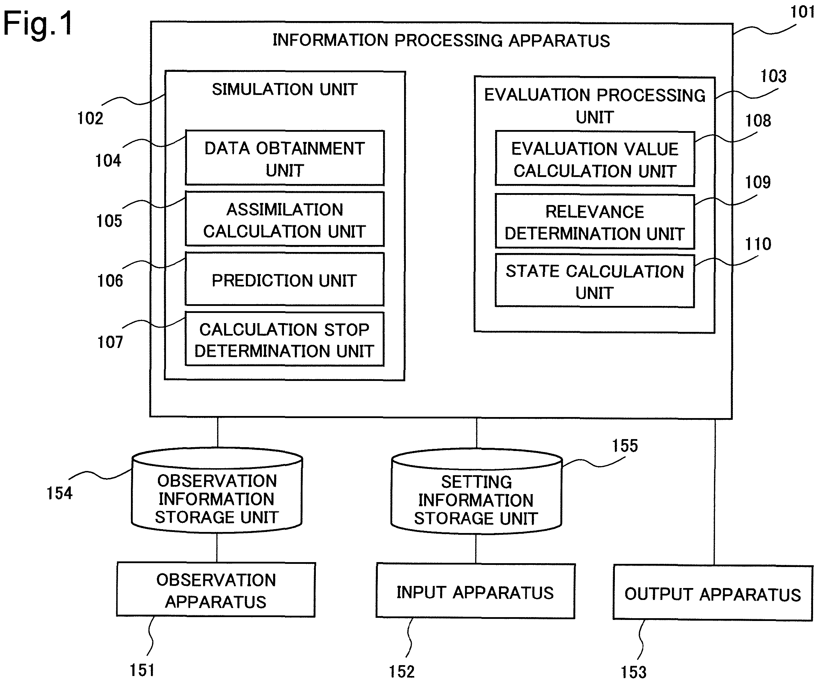

A configuration of an information processing apparatus 101 according to a first example embodiment of the present invention will be described in detail with reference to Fig. 1. Fig. 1 is a block diagram illustrating a configuration of the information processing apparatus 101 according to the first example embodiment of the present invention.

The information processing apparatus 101 broadly includes a simulation unit (simulator) 102 and an evaluation processing unit (evaluation processor) 103. The simulation unit 102 includes a data obtainment unit (data obtainer) 104, an assimilation calculation unit (assimilation calculator) 105, a prediction unit (predicator) 106, and a calculation stop determination unit (calculation stop determiner) 107. The evaluation processing unit 103 includes an evaluation value calculation unit (evaluation value calculator) 108, a relevance determination unit (relevance determiner) 109, and a state calculation unit (state calculator) 110.

An observation apparatus 151 observes a state of a target, generates observation information (observation value) representing the observed state, and stores the generated observation information in an observation information storage unit 154. An input apparatus 152 inputs setting information from outside and stores the input setting information in a setting information storage unit 155. Setting information includes information representing a setting model for a target, and a stopping criterion representing whether or not to stop an assimilation process. The stopping criterion is a criterion for that a timing in a process being performed reaches a predetermined timing (i.e., stopping timing). Alternatively, the stopping criterion may be a criterion for that any one of state values satisfies a predetermined criterion (e.g., a state value exceeds a threshold value) among state values (state information). A state value (state information) represents a state of a target. State information is information representing a remarkable state among states of a target crop, an expressway, or the like. In this case, the state information is information representing a yield of a later-described target crop, a time required for elimination of congestion, or the like.

The information processing apparatus 101 is connected (or communicably connected) to the observation information storage unit 154, the setting information storage unit 155, and an output apparatus 153. The information processing apparatus 101 can read observation information stored in the observation information storage unit 154, and setting information stored in the setting information storage unit 155. The information processing apparatus 101 may output a result of a process to the output apparatus 153.

Each component in the information processing apparatus 101 according to the first example embodiment will be described.

The data obtainment unit 104 reads observation information stored in the observation information storage unit 154, and outputs the read observation information to the assimilation calculation unit 105.

The assimilation calculation unit 105 inputs the observation information output by the data obtainment unit 104. Then, the assimilation calculation unit 105 generates a plurality of scenarios (particles). This process will be described.

First, the assimilation calculation unit 105 generates a plurality of sets including state information (state value) at a certain timing. In other words, the assimilation calculation unit 105 generates a plurality of scenarios (particles) including the state information (state value). Then, the assimilation calculation unit 105 calculates random numbers having a certain dispersion (variance) around observation information, and generates a plurality of sets constructed by combining the generated random numbers. The assimilation calculation unit 105 generates a scenario (particle) including the set. Therefore, the above-described process is a process of replicating, changing, or deleting a particle in such a way as to generate a probability distribution for generating observation information at a certain timing. By the above-described process, the assimilation calculation unit 105 executes a filtering process based on observation information at each timing and a mathematical model. Moreover, based on input observation information, the assimilation calculation unit 105 calculates at least one likelihood among likelihoods (a degree of a possibility) as described above with reference to Eqns. 1 to 4 and average likelihoods (a degree of a possibility averaged on a scenario) as described above with reference to Eqns. 5 to 8. The assimilation calculation unit 105 outputs, to the evaluation processing unit 103, the likelihood and the average of likelihood that have been calculated.

Then, the assimilation calculation unit 105 outputs a plurality of generated particles to the prediction unit 106. In relation to a timing at which observation information is not observed, the assimilation calculation unit 105 outputs a particle to the prediction unit 106 without performing the above-described filtering process.

The prediction unit 106 inputs a particle (scenario) output by the assimilation calculation unit 105 and generates state information at a timing (t+1) based on state information at the timing t in the input scenario and a mathematical model obtained by discretizing a differential equation representing an aspect of change of a state of a target along with time transition.

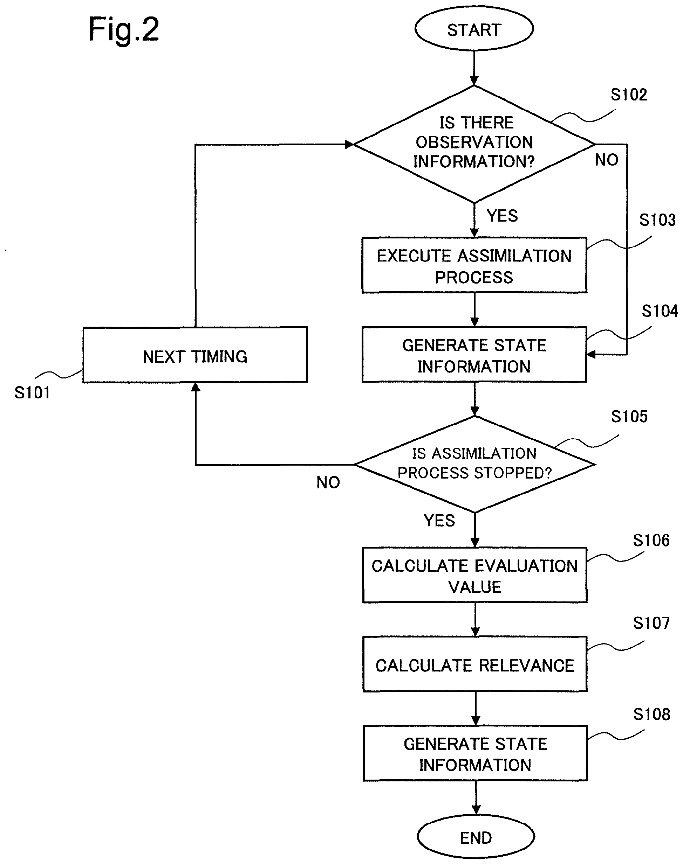

The calculation stop determination unit 107 reads a stopping criterion for determining whether or not to stop calculation out of setting information stored in the setting information storage unit 155. The calculation stop determination unit 107 determines whether or not to stop an assimilation process based on the read stopping criterion. When determining to stop the assimilation process, the calculation stop determination unit 107 stops the assimilation process. When determining not to stop the assimilation process, the calculation stop determination unit 107 executes a process illustrated in a step S101 (described later with reference to Fig. 2).

The simulation unit 102 outputs, to the evaluation processing unit 103, a likelihood (exemplified by Eqns. 1 to 8) calculated in a process of executing an assimilation process, and state information of each particle calculated as a result of executing the assimilation process. Therefore, the simulation unit 102 estimates a scenario representing an aspect of change of a state of a target by executing the above-described process.

In the evaluation processing unit 103, the evaluation value calculation unit 108 inputs state information output by the simulation unit 102 and a likelihood. The evaluation value calculation unit 108 calculates an evaluation value (Eqn. 9) representing a degree of similarity between a particle (scenario) and a state actually occurring in a target by executing predetermined evaluation processing for the input likelihood.

$$E^{(i)} = \sum_j \alpha_j \lambda_{j,t_1 \to t_2}^{(i)} \qquad \bullet \quad \bullet \quad \bullet \left(\text{Eqn.}9\right)$$

$\sum_j$ represents calculating a summation relating to j. $\alpha_j$ represents weight. $\sum_j \alpha_j = 1$.

The evaluation value exemplified by Eqn. 9 is an expectation value of a likelihood on each scenario, and therefore, is information representing a degree of a likelihood (possibility). Therefore, as an evaluation value for a particle is a greater value, the particle represents a state closer to an actually occurring state. As an evaluation value for a particle is a smaller value, the particle represents a state far from an actually occurring state. A predetermined evaluation process is, for example, a process of calculating an evaluation value in accordance with Eqn. 9.

The evaluation value calculation unit 108 outputs a calculated evaluation value to the relevance determination unit 109. The evaluation value calculation unit 108 executes the above-described process for each particle.

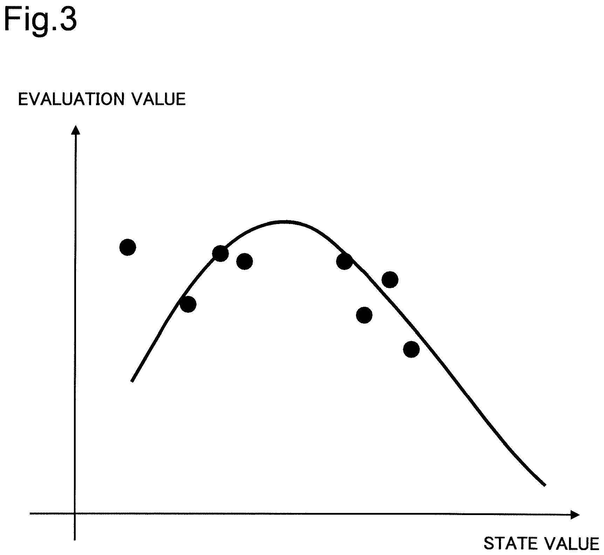

The relevance determination unit 109 inputs the evaluation value output by the evaluation value calculation unit 108 for each particle. As exemplified in Fig. 3 (described later), the relevance determination unit 109 acquires a relevance in which at least one value of state information in each particle and an evaluation value for the particle are suitable to each other. For example, the relevance determination unit 109 executes, for a plurality of particles, a fitting process of acquiring a relevance in which the value and the evaluation value are suitable to each other. The fitting process is a process of acquiring a coefficient in a predetermined relevance, based on the value and the evaluation value. A predetermined relevance is, for example, a Gaussian function, a quadratic function, or a composite function in which these functions are added together. When a predetermined relevance is a convex function, searching for a great value in the relevance can achieve acquiring a value having a high evaluation value.

The relevance determination unit 109 may execute the above-described fitting process for a value in case that state information satisfies a predetermined constraint criterion. For example, in case of a simulation of a process of growing a crop, the relevance determination unit 109 may execute the above-described fitting process for only state information satisfying a predetermined constraint condition of "a range from 50 tons to 150 tons per hectare" for a yield x of a crop being one of state information. This can be applied to, for example, a case where an amount of a harvestable crop per hectare can be acquired in advance. The relevance determination unit 109 outputs a calculated relevance to the state calculation unit 110. A fitting process will be described later with reference to Fig. 3.

The state calculation unit 110 inputs a relevance output by the relevance determination unit 109, generates estimation information of state information based on the input relevance and outputs the generated estimation information to the output apparatus 153. In this process, the state calculation unit 110 acquires, as the estimation information, state information (e.g., high possible state information) in case that a possibility satisfies a predetermined selection criterion in the input relevance. For example, when a predetermined selection criterion is a criterion that a possibility is a great value in the relevance, the state calculation unit 110 acquires, as the estimation information, state information in case that an evaluation value representing a possibility is a great value in the input relevance. In this case, the state calculation unit 110 acquires state information in case that the evaluation value is a great value by mutually comparing evaluation values included in the input relevance. For example, the state calculation unit 110 may acquire, as the estimation information, state information in case that an evaluation value in the relevance is highest (or substantially highest). A substantially highest value has only to be, for example, a value within a predetermined range (e.g., 3%, 5%, or 10%) from a highest value.

Next, a fitting process will be described with reference to Fig. 3. Fig. 3 is a diagram conceptually illustrating a relevance acquired by a fitting process based on a state value relating to a state in a scenario and an evaluation value for the scenario. A horizontal axis of Fig. 3 represents a remarkable state value in state information, and represents that the state value is greater when being closer to a right side, and the state value is smaller when being closer to a left side. A vertical axis of Fig. 3 represents an evaluation value, and represents that an evaluation value is greater (i.e., more likely) when being closer to an upper side, and an evaluation value is smaller (i.e., less likely) when being closer to a lower side. A black point represents a position determined by an evaluation value for a scenario and a value of a remarkable state in state information in the scenario. A curve represents a relevance acquired based on a position determined for each particle. A fitting process can obtain a relevance being less divergent from a position determined for each particle.

In a fitting process, a process may be executed without using information about a position divergent from a relevance (i.e., an outlier). In this case, the process is a process of suppressing an influence of an outlier on a value of a parameter in a relevance. The process of suppressing an influence of an outlier may be, for example, a process which does not refer to a position being significantly divergent from a distribution of a remarkable position. A determination process of whether or not to be divergent is executed, for example, by determining whether or not the position is "$3 \times \sigma$" ($\sigma$ represents a standard deviation) or more away from an average position of all positions. In this case, a determination process of whether or not to be divergent is executed, for example, by determining whether or not the position is within a predetermined distance from an average position of all positions. Alternatively, the process of suppressing an influence of the outlier may be a process based on a predetermined constraint condition as described above. In the present example embodiment, a distance represents a mathematical distance.

The process of suppressing an influence of the outlier may be, for example, a process of selecting an outlier depending on a cluster acquired in accordance with a clustering method of a plurality of positions. A method for clustering is, for example, a K-means method or an X-means method. In this case, the process of suppressing an influence of the outlier includes acquiring a cluster by clustering a plurality of positions and calculating a number of positions belonging to an acquired cluster. The process includes classifying the acquired cluster into a cluster including a large number of positions and a cluster including a small number of positions and selecting a position belonging to the latter cluster as an outlier. According to the process, estimation information can be more accurately acquired by a process of suppressing an influence of an outlier.

Next, a process in the information processing apparatus 101 according to the first example embodiment of the present invention will be described in detail with reference to Fig. 2. Fig. 2 is a flowchart illustrating flow of a process in the information processing apparatus 101 according to the first example embodiment.

The data obtainment unit 104 determines whether or not observation information is stored in the observation information storage unit 154 (step S102). When observation information is stored in the observation information storage unit 154 (YES in the step S102), the data obtainment unit 104 acquires observation information from the observation information storage unit 154, and outputs observed observation information to the assimilation calculation unit 105. The assimilation calculation unit 105 inputs observation information output by the data obtainment unit 104. The assimilation calculation unit 105 executes an assimilation process (step S103) and a filtering process as described above. In an assimilation process, the assimilation calculation unit 105 calculates, based on the acquired observation information, at least one likelihood among likelihoods as described above with reference to Eqns. 1 to 8.

When observation information is not stored in the observation information storage unit 154 (NO in the step S102), the assimilation process illustrated in a step S103 is not executed. In this case, the data obtainment unit 104 outputs, to the prediction unit 106, a signal representing that observation information is absent.

The prediction unit 106 inputs state information output by the assimilation calculation unit 105, and a likelihood, or a signal output by the data obtainment unit 104. Based on input state information and a mathematical model, the prediction unit 106 generates state information at a timing next to a timing relating to the input state information (step S104).

The calculation stop determination unit 107 reads a stopping criterion for determining whether or not to stop an assimilation process out of setting information stored in the setting information storage unit 155, and determines whether or not to stop an assimilation process based on the read stopping criterion (step S105). When determining to stop an assimilation process (YES in the step S105), the calculation stop determination unit 107 stops the assimilation process. When determining not to stop the assimilation process (NO in the step S105), the calculation stop determination unit 107 executes a process (step S101) of putting a timing ahead. After a process illustrated in the step S101, a process illustrated in a step S102 is executed.

In case of YES in the step S105, the evaluation value calculation unit 108 executes a predetermined evaluation process for the third likelihood (exemplified by Eqn. 3) of each particle calculated by the assimilation calculation unit 105, and state information of each particle calculated by the prediction unit 106. By this process, the evaluation value calculation unit 108 calculates an evaluation value (exemplified by Eqn. 9) representing a degree at which a particle (scenario) is similar to a state actually occurring in a target (step S106).

Then, the relevance determination unit 109 acquires a relevance suitable to an evaluation value (exemplified by Eqn. 9) calculated by the evaluation value calculation unit 108 for each particle and state information calculated by the prediction unit 106 for the particle (step S107). For example, the relevance determination unit 109 acquires the relevance by executing a process of fitting as exemplified by Fig. 3.

The evaluation value calculation unit 108 generates state information in case that an evaluation value is great based on a relevance acquired by the relevance determination unit 109 (step S108). The evaluation value calculation unit 108 generates state information in case that an evaluation value is great, for example, by acquiring state information in case that a Gaussian function is maximum.

Next, an advantageous effect of the information processing apparatus 101 according to the first example embodiment of the present invention will be described.

The information processing apparatus 101 according to the first example embodiment is able to provide information serving as a basis for accurately estimating a state of a target. A reason for this is that, by determining a state giving a likely state, based on a qualitative relevance between an evaluation value of a particle (scenario) relating to a target and state information about the target, it is possible to reduce a possibility that prediction divergent from observation information for the target is executed, as described above. A reason for this will be specifically described.

Maximum likelihood estimation includes generating state information in a particle (scenario) having a greatest likelihood as optimum estimation information. Moreover, a particle as described above discretely appears in a solution space. Thus, in likelihood estimation, density of particles in the solution space is increased by increasing particles in the solution space for a purpose of calculating a precise particle. As a result, in likelihood estimation, a calculation amount increases when a precise particle is calculated.

For example, when a target is a yield of a crop, a solution space represents one coordinate axis relating to the yield. A yield in each particle (scenario) is distributed on the coordinate axis (e.g., on a coordinate axis indicated by "value of state" in Fig. 3). However, as described above about the problem found out by the inventor of the present application, a value (e.g., a coordinate value of a black point in a horizontal axis direction of Fig. 3) distributed on the coordinate axis does not necessarily represent a state which actually occurs in a target.

The information processing apparatus 101 according to the present example embodiment acquires a relevance (a curve in Fig. 3) between an evaluation value calculated for each scenario and state information representing a remarkable state in the scenario, and acquires likely state information in accordance with the acquired relevance. In this case, the information processing apparatus 101 generates optimum estimation information, for example, by determining a value of a parameter in a predetermined relevance, based on the evaluation value and the state information (i.e., a position of a black point in Fig. 3). A predetermined relevance is, for example, continuous in a section in which state information is distributed. As a result, there is a high possibility that a solution acquired by the information processing apparatus is a state which actually occurs in a target, as compared with a case where discrete state information is processed.

Furthermore, by using, as a predetermined relevance, a relevance such as a Gaussian function or a convex function relating to a likelihood, an outlying set can be removed from a distribution of a set of the evaluation value and the state information. As a result, a state having a low possibility of actually occurring in a target (i.e., an unrealistic state) can be excluded from the solution space. In consequence, the information processing apparatus according to the present example embodiment is able to provide information serving as a basis for more accurately estimating a state of a target.

Still further, the information processing apparatus 101 according to the present example embodiment acquires a relevance suitable to an evaluation value and state information, and acquires state information having a great evaluation value in accordance with the acquired relevance. Therefore, it is not necessary to generate a huge number of particles for a purpose of providing information serving as a basis for accurately estimating a state of a target. In contrast, the apparatus disclosed in PTL 1 or PTL 2 selects state information having a high evaluation value, and therefore, it is necessary to generate a huge number of particles for a purpose of providing information serving as a basis for accurately estimating a state of a target.

Further yet, while a process in the information processing apparatus 101 according to the present example embodiment is described with reference to an assimilation process as an example, a process in the information processing apparatus 101 is not necessarily a process relating to an assimilation process. A process in the information processing apparatus 101 has only to be executed in relation to a process of predicting a state of a target, and further analyzing a dependence relation between the predicted state and observation information observed for the target, and is not limited to the above-described example. Each of the following example embodiments is not limited to an assimilation process either.

<Second example embodiment>

Next, a second example embodiment of the present invention based on the above-described first example embodiment will be described.

In the following description, a characteristic part according to the present example embodiment will be mainly described, and overlapping description will be omitted by assigning a same reference sign to a component similar to that in the first example embodiment.

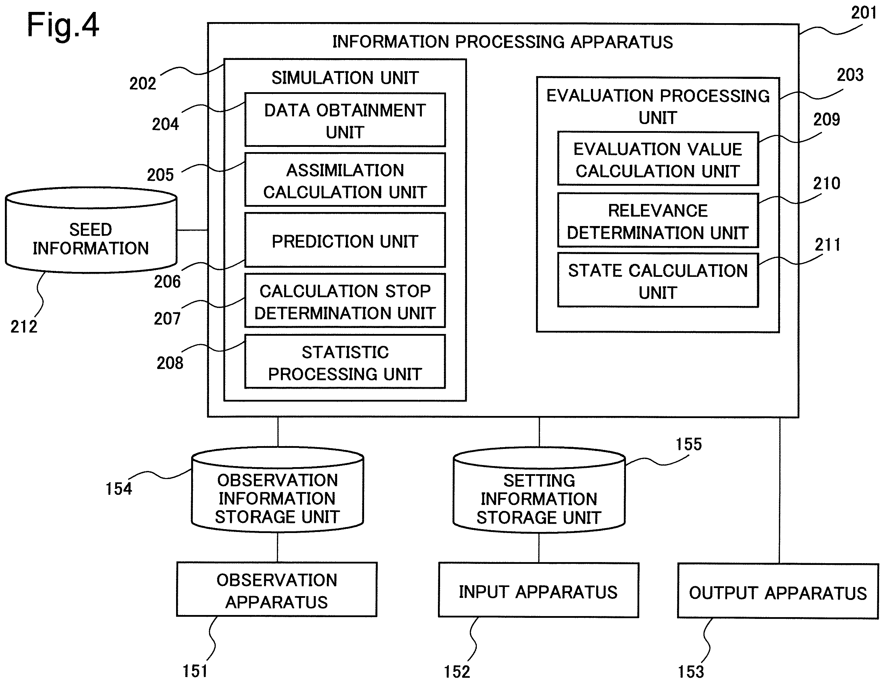

A configuration of an information processing apparatus 201 according to the second example embodiment of the present invention will be described in detail with reference to Fig. 4. Fig. 4 is a block diagram illustrating a configuration of the information processing apparatus 201 according to the second example embodiment of the present invention.

The information processing apparatus 201 broadly includes a plurality of simulation units (simulators) 202 and an evaluation processing unit (evaluation processor) 203. The simulation units 202 each include a data obtainment unit (data obtainer) 204, an assimilation calculation unit (assimilation calculator) 205, a prediction unit (predictor) 206, a calculation stop determination unit (calculation stop determiner) 207, and a statistic processing unit (statistic processor) 208. The evaluation processing unit 203 includes an evaluation value calculation unit (evaluation value calculator) 209, a relevance determination unit (relevance determiner) 210, and a state calculation unit (static calculator) 211.

The data obtainment unit 204 has a function similar to the function of the data obtainment unit 104. The assimilation calculation unit 205 has a function similar to the function of the assimilation calculation unit 105. The prediction unit 206 has a function similar to the function of the prediction unit 106. The calculation stop determination unit 207 has a function similar to the function of the calculation stop determination unit 107. The evaluation value calculation unit 209 has a function similar to the function of the evaluation value calculation unit 108. The relevance determination unit 210 has a function similar to the function of the relevance determination unit 109. The state calculation unit 211 has a function similar to the function of the state calculation unit 110. Therefore, the simulation unit 202 estimates a scenario representing an aspect of change of a state of a target.

An observation apparatus 151 observes a state of a target, generates observation information (observation value) representing the observed state, and stores the generated observation information in an observation information storage unit 154. An input apparatus 152 inputs setting information from outside and stores the input setting information in a setting information storage unit 155. Setting information includes information representing a setting model for a target and a stopping criterion representing whether or not to stop an assimilation process. The stopping criterion is a criterion for determining that timing at which a process is being performed reaches a predetermined timing (i.e., stopping timing). Alternatively, the stopping criterion may be a criterion that any one of state values in state information satisfies a predetermined criterion (e.g., a state value exceeds a threshold value).

The information processing apparatus 201 is connected (or communicably connected) to the observation information storage unit 154, the setting information storage unit 155, and an output apparatus 153. The information processing apparatus 201 can read observation information stored in the observation information storage unit 154 and setting information stored in the setting information storage unit 155. The information processing apparatus 201 may output a result of a process to the output apparatus 153.

In the information processing apparatus 201 according to the present example embodiment, the simulation unit 202 reads observation information, setting information, and a random seed being an initial condition for generating pseudo-random numbers. In the simulation unit 202, the assimilation calculation unit 205 reads a random seed from, for example, random seed information 212 and generates pseudo-random numbers based on the read random seed. The assimilation calculation unit 205 may read pseudo-random numbers or random numbers instead of a random seed. Hereinafter, pseudo-random numbers or random numbers are generically referred to as "random numbers". The random numbers are used when an initial state value is calculated, or when a particle is redistributed. Moreover, the assimilation calculation unit 205 reads a plurality of random seeds differing in accordance with the simulation units 202. The assimilation calculation unit 205 executes an assimilation process and a filtering process as described in the first example embodiment. In the assimilation process, the assimilation calculation unit 205 calculates at least one of likelihoods (a degree of a possibility and a degree of a likelihood) as described above with reference to Eqns. 1 to 8.

The statistic processing unit 208 selects a representative likelihood and representative state information by statistically processing a likelihood and state information of each particle calculated by the assimilation calculation unit 205 (step S203). As indicated by Eqn. 10, the statistic processing unit 208 calculates, for example, a weighted average in which state information in a scenario represented by each particle is weighted by a fourth likelihood (Eqn. 4) for the particle, as representative state information $x_{kl}$ (however, k is a natural number indicating a random seed. 1 represents first state information).

$$x_{kl} = \frac{\sum_i \lambda_{t_1 \to t_2}^{(i)} x_{kl}^{(i)}}{\sum_i \lambda_{t_1 \to t_2}^{(i)}} \quad \cdots \quad (\text{Eqn.}10)$$

However, $x_{kl}^{(i)}$ represents a value of l-th state (variable) calculated based on pseudo-random numbers generated according to a k-th random seed for an i-th particle.

In other words, the statistic processing unit 208 calculates average state information in accordance with a process indicated by Eqn. 10.

The statistic processing unit 208 further calculates, for example, a weighted average in which a likelihood relating to a scenario represented by each particle is weighted by the fourth likelihood (Eqn. 4) relating to the particle, as a representative likelihood $L_{kj}$ (k is a natural number indicating a random seed. j represents j-th state information) relating to observation information.

$$L_{kj} = \frac{\sum_i \lambda_{t_1 \to t_2}^{(i)} L_{kj}^{(i)}}{\sum_i \lambda_{t_1 \to t_2}^{(i)}} \quad \cdots \quad (\text{Eqn.}11)$$

$L_{kj}^{(i)}$ represents an average likelihood for a j-th state (variable) calculated based on pseudo-random numbers generated in accordance with a k-th random seed for an i-th particle. For example, $L_{kj}^{(i)}$ represents a likelihood calculated according to Eqn. 7.

In other words, the statistic processing unit 208 calculates an average of likelihoods (i.e., an average likelihood) in accordance with a process indicated by Eqn. 11.

Each of the simulation units 202 outputs, to the evaluation value calculation unit 209, the representative likelihood and representative state information that have been calculated in accordance with a process indicated by Eqns. 10, 11, and the like. Therefore, each of the simulation units 202 calculates the above-described value not for each particle but for each random seed for calculating each state process.

The relevance determination unit 210 and the state calculation unit 211 execute a process similar to the process described in the first example embodiment. The state calculation unit 211 outputs the generated estimation information to the output apparatus 153.

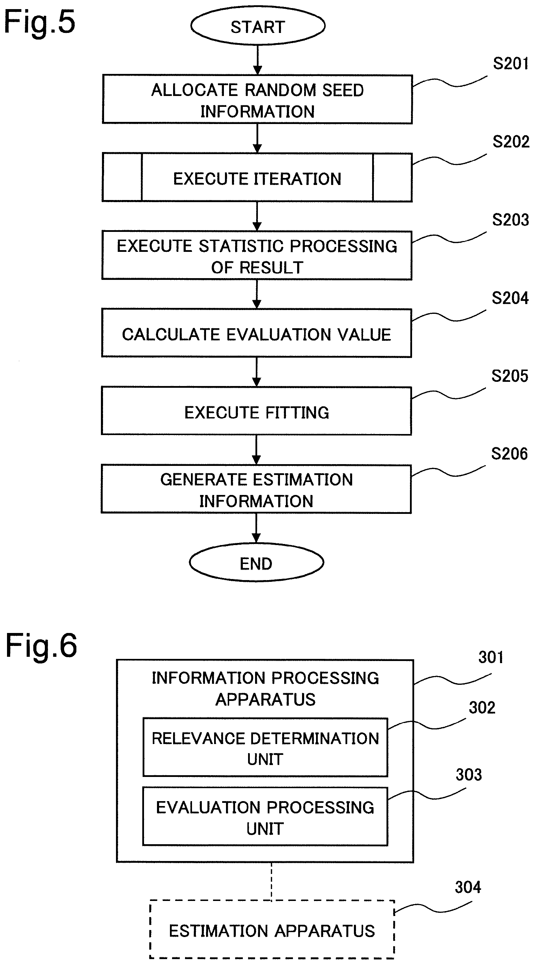

Next, a process in the information processing apparatus 201 according to the second example embodiment of the present invention will be described in detail with reference to Fig. 5. Fig. 5 is a flowchart illustrating flow of a process in the information processing apparatus 201 according to the second example embodiment.

Each of the simulation units 202 reads a random seed from random seed information. Each of the simulation units 202 executes the above-described process with reference to a step S201 and a step S202 in a parallel, pseudo-parallel, or sequential way. The step S202 represents a process illustrated in the steps S101 to S105 in Fig. 2. A process executed in the step S202 will be specifically described with reference to Fig. 2.

In each of the simulation units 202, the data obtainment unit 204 determines whether or not observation information is stored in the observation information storage unit 154 (step S102). When observation information is stored in the observation information storage unit 154, the assimilation calculation unit 205 executes an assimilation process as described above, based on the observation information (step S103). In the assimilation process, the assimilation calculation unit 205 calculates, based on the observation information, at least one likelihood among likelihoods as described with reference to Eqns. 1 to 8. When observation information is not stored in the observation information storage unit 154 (NO in the step S102), the assimilation calculation unit 205 does not execute a process indicated in a step S103.

When observation information is not stored in the observation information storage unit 154 (NO in the step S102), the prediction unit 206 generates state information relating to a state at a next timing, based on a mathematical model (step S104). When observation information is stored in the observation information storage unit 154 (YES in the step S102), the prediction unit 206 generates state information about a state at a next timing, in relation to a calculated particle (scenario) (step S104).

The process in the steps S101 to S105 (Fig. 2) is repeatedly executed when a predetermined stopping criterion is not satisfied (NO in the step S105). When a predetermined stopping criterion is satisfied (YES in the step S105), the simulation unit 202 generates a representative likelihood and representative state information by executing a process described with reference to Eqns. 10 and 11 (step S203 in Fig. 5).

After all the simulation units 202 generate representative likelihoods and representative state information, the evaluation value calculation unit 209 calculates an evaluation value for each random seed, based on the representative likelihood and the representative state information (step S204). An evaluation value is, for example, a value in which a representative likelihood is averaged ono each scenario.

The relevance determination unit 210 executes a fitting process relating to the evaluation value calculated for each random seed for a particle (scenario) and specified state information out of representative state information in the scenario (step S205). As illustrated in Fig. 3, a fitting process is a process of acquiring a relevance by, for example, calculating a value of a parameter in a predetermined relevance such as a Gaussian function.

The state calculation unit 211 generates, as estimation information, a state value in case that an evaluation value is highest in relation to the acquired relevance (step S206) and outputs the generated estimation information to the output apparatus 153.

In the above-described process, a number of random numbers being a basis for calculating an evaluation value as indicated by Eqn. 9 may be adjusted based on a fitting degree of the calculated relevance, and information being a basis of calculating a parameter in the relevance (i.e., a combination of an evaluation value and predetermined state information). For example, the relevance determination unit 210 calculates a fitting degree as described above in a process of acquiring a relevance, and determines whether or not to further generate a particle, based on the acquired fitting degree. For example, when a fitting degree is low, the relevance determination unit 210 determines to further generate a particle. When the relevance determination unit 210 determines to generate a particle, the simulation unit 202 further generates a particle.

Furthermore, although a fitting process is executed based on representative state information and a representative likelihood relating to each random seed in the above-described process, a fitting process may be executed without referring to representative state information and a representative likelihood. In this case, the relevance determination unit 210 executes a fitting process, based on a likelihood for each particle calculated in accordance with each random seed, and state information in the particle.

Alternatively, the relevance determination unit 210 may calculate, as a representative likelihood or representative state information, a likelihood and state information in case that a likelihood for each random seed is highest.

Next, when the above-described process is applied to agriculture, a process executed by the information processing apparatus 201 according to the present example embodiment will be described.

First, when the information processing apparatus 201 according to the present example embodiment is applied to a simulation relating to growth of a plant being a target crop, a process executed by the information processing apparatus 201 will be described with reference to Figs. 2 and 5.

It is assumed that observation information observed by the observation apparatus 151 being a soil water amount sensor, a leaf area index sensor, a plant extension sensor, an in-leaf nitrogen concentration sensor, or the like observing in relation to a plant is stored in the observation information storage unit 154 for each observation date.

The data obtainment unit 204 reads observation information stored in the observation information storage unit 154.

For convenience of description, it is assumed that a period in which the plant sprouts and then fruit of the plant ripens (i.e., a target crop can be harvested) is 200 days. In a simulation, it is assumed that a state of a plant during the 200 days is simulated. Moreover, it is assumed that a state to which attention is paid is total weight of fruit on the 200th day. In this case, a stopping criterion set in the setting information storage unit 155 is, for example, a criterion that a timing is 200 days or more. A plant growth model is information representing a relevance between observation information and information representing a state of a plant.

The assimilation calculation unit 205 generates 1000 particles (scenarios), for example, by using pseudo-random numbers generated in accordance with a random seed. Based on a state in the generated particle, a plant growth model, and observation information observed in relation to the plant, the assimilation calculation unit 205 executes an assimilation process (step S103 in Fig. 2) as described above in the first example embodiment. When observation information relating to a timing is not stored in the observation information storage unit 154 (NO in the step S102), the assimilation calculation unit 205 does not execute the assimilation process (step S103).

Then, the prediction unit 206 estimates state information at a next timing relating to the plant by applying a plant growth model to state information in a particle calculated by the assimilation calculation unit 205 (step S104). In other words, the prediction unit 206 generates state information at a next timing.

When a predetermined stopping criterion (in this example, a criterion that a timing is 200 days or more) is not satisfied (NO in the step S105), a process in steps S101 to S105 (Fig. 2) is executed for a next timing. When a timing is 200 days or more (YES in the step S105), the simulation unit 202 stops an assimilation process.

By executing an assimilation process for input data as described above, the simulation unit 202 calculates likelihoods for 1000 respective particles (e.g., likelihoods $(\lambda_{j,0\to200}^{(i)})$ for a soil water amount, a leaf area index, extension, and in-leaf nitrogen concentration relating to a plant, and state information relating to the particles (e.g., total weight of fruit $x^{(i)}$).

However, a timing representing a sprouting timing is represented as "0", and a timing representing 200 days later is represented as "200".

For example, when each reliability of the soil water amount sensor, the leaf area index sensor, the plant extension sensor, and the in-leaf nitrogen concentration sensor is $\alpha_j$ (j is a natural number. j represents an observation apparatus), the evaluation value calculation unit 209 calculates an evaluation value of each particle in accordance with Eqn. 12 (step S204 in Fig. 5).

$$E^{(i)} = \sum_j \alpha_j \lambda_{j,0\to200}^{(i)} \qquad \bullet \quad \bullet \quad \bullet \text{(Eqn.12)}$$

In accordance with a predetermined relevance as indicated by Eqn. 13, the relevance determination unit 210 determines parameters $(\mu, \sigma)$ in the predetermined relevance.

$$E = \frac{1}{\sqrt{2\pi\sigma^2}} \exp(-\frac{(x-\mu)^2}{2\sigma^2}) \qquad \bullet \quad \bullet \quad \bullet \text{(Eqn.13)}$$

$\mu$ represents an average value of x. $\sigma^2$ represents a dispersion of x. $\pi$ represents a ratio of the circumference of a circle to its diameter. exp represents an index function of a Napier's constant. x represents a parameter (variable) relating to total weight. E represents a parameter (variable) relating to an evaluation value $E^{(i)}$ indicated by Eqn. 12.

Therefore, the relevance determination unit 210 executes a fitting process by calculating the parameters $(\mu, \sigma)$ in a relevance as indicated by Eqn. 13 (step S205). Based on a relevance calculated by the simulation unit 202, the state calculation unit 211 acquires a state value (i.e., $\mu$) in case that an evaluation value in the relevance is highest (step S206).

For example, it is assumed that an average of a state value (in this example, total weight of fruit of a plant) being a target is 130 tons per hectare (i.e., 130 (t/ha)), total weight of a crop in a particle having a highest likelihood is 125 (t/ha), and total weight in a particle having a second highest likelihood is 134 (t/ha). In the maximum likelihood estimation method described in PTL 1 and the like, 125 (t/ha) being a state value in a particle having a highest likelihood is calculated. In contrast, the information processing apparatus 201 according to the present example embodiment calculates 129(=(134+125)÷2)(t/ha) as estimation information relating to a state value by calculating a weight-averaged likelihood as described above for all particles. Based on observation information relating to a plant, and a plant growth model relating to the plant, the information processing apparatus 201 estimates that total weight of fruit of the plant on the 200th day from sprouting of the plant is 129 tons per hectare.

The state calculation unit 211 outputs generated estimation information to the output apparatus 153 being a display or the like.

Next, a process in the information processing apparatus 201 in case that the information processing apparatus 201 according to the present example embodiment is applied to a simulation for predicting congestion of vehicles will be described.

The observation information storage unit 154 stores observation information observed by the observation apparatus 151 being a traffic counter, a number-of-vehicle sensor installed at an entrance/exit of each expressway, or the like. Observation information is, for example, information observed every 5 minutes. Observation information is, for example, information representing a position where congestion is occurring, a flow speed of a vehicle at each position, density of vehicles, a number of vehicles flowing into each expressway, and a number of vehicles flowing out of an expressway.

For convenience of description, it is assumed that a simulation is a simulation of congestion prediction. In the simulation, it is assumed that congestion for three hours from present is simulated. For example, in the simulation, a time required for elimination of congestion (hereinafter, referred to as an "elimination time") is estimated at each position where congestion occurs. A predetermined stopping criterion is a criterion that a timing elapses three hours from present. It is assumed that specified state information is a time required for elimination of congestion. A process will be described with reference to Figs. 2 and 5.

The data obtainment unit 204 determines whether or not observation information is stored in the observation information storage unit 154 (step S102). When observation information is stored in the observation information storage unit 154 (YES in the step S102), the data obtainment unit 204 reads observation information from the observation information storage unit 154, and outputs the read observation information to the assimilation calculation unit 205. When observation information is not stored in the observation information storage unit 154 (NO in the step S102), the data obtainment unit 204 outputs, to the prediction unit 206, a request signal for generating state information at a next timing.

The assimilation calculation unit 205 inputs observation information output by the data obtainment unit 204. Based on the input observation information and a congestion model, the assimilation calculation unit 205 generates 1000 particles (i.e., scenarios), for example, by using pseudo-random numbers generated according to a random seed. The assimilation calculation unit 205 generates 1000 particles by executing, based on observation information, an assimilation process for the generated 1000 particles (step S103). In the assimilation process, the assimilation calculation unit 205 calculates, based on the observation information, at least one likelihood among likelihoods (a degree of a possibility and a degree of a likelihood) as described with reference to Eqns. 1 to 8.

Based on state information in an updated particle and the congestion model, the prediction unit 206 predicts state information at a next timing (step S104). A process illustrated in the steps S101 to S105 (Fig. 2) is executed for each timing in a period not satisfying a predetermined stopping criterion (NO in the step S105).

When the calculation stop determination unit 207 determines that a predetermined stopping criterion is satisfied (i.e., when three hours, which is a time in a simulation, elapse from present, YES in the step S105), the simulation unit 202 stops an assimilation process.

By executing a process as described above, the simulation unit 202 calculates a likelihood for each of 1000 particles, and state information for the particle. The likelihood is observation information in the particle, and is, for example, a likelihood ($\lambda_{j,0\to3h}^{(i)}$) relating to information such as a position of congestion occurrence, a speed of a vehicle moving at each position, density of vehicles, a number of vehicles flowing into each expressway, or a number of vehicles flowing out of each expressway. State information is, for example, a time ($x_k^{(i)}$) required for elimination of congestion at a k-th position being specified state information.

It is assumed that a present timing is represented as "0", and a timing of three hours later is represented as "3h"

Based on a likelihood calculated by the simulation unit 202 for each particle and state information relating to the particle, the evaluation value calculation unit 209 calculates an evaluation value relating to the particle (step S204). For example, when an error of observation information such as a position of congestion occurrence, a flow speed of a vehicle at each position, density of vehicles, a number of vehicles flowing into each expressway, or a number of vehicles flowing out of the expressway is $\alpha_j$, an evaluation value relating to each particle is calculated as indicated by Eqn. 14.

$$E^{(i)} = \sum_j \alpha_j \lambda_{j,0\to3h}^{(i)} \quad \cdot \cdot \cdot \left(\text{Eqn.} 14\right)$$

When a predetermined relevance is represented by Eqn. 15, the relevance determination unit 109 acquires, based on a relevance indicated by Eqn. 15, values of parameters ($\sigma$, $\mu$) included in the relevance.

$$E = \frac{1}{\sqrt{2\pi\sigma^2}} \exp(-\frac{(x-\mu)^2}{2\sigma^2}) \quad \cdot \cdot \cdot (\text{Eqn} 15)$$

$\mu$ represents an average value of x. $\sigma^2$ represents a variance of x. $\pi$ represents a ratio of the circumference of a circle to its diameter. exp represents an index function of a Napier's constant. x represents a parameter (variable) of a time required for elimination of congestion. E represents a parameter (variable) relating to an evaluation value $E^{(i)}$ indicated by Eqn. 14.

Therefore, the relevance determination unit 109 calculates a relevance between observation information in a particle and an evaluation value relating to the particle by acquiring values of parameters ($\sigma$, $\mu$). In other words, the relevance determination unit 109 executes a fitting process by calculating values of the parameters (step S205).

Based on a relevance acquired by the relevance determination unit 109, the state calculation unit 211 calculates, as estimation information (i.e., an estimation value $\mu$), state information (e.g., value information of x) in case that a value of a relevance indicated by Eqn. 15 is highest in the acquired relevance (step S206).

For example, it is assumed that state information (in this example, a time required for elimination of congestion) relating to a k-th position having a highest likelihood among times required for elimination of congestion occurring at a k-th position (k is a natural number) is 2.3 hours. Moreover, it is assumed that state information (in this example, a time required for elimination of congestion) relating to a k-th particle having a second highest likelihood is 1.8 hours.

In this case, the apparatus presented in PTL 1 and the like calculates, as estimation information, state information relating to 2.3 hours in case that a likelihood is highest. In contrast, the information processing apparatus 201 according to the present example embodiment calculates, as estimation information, state information relating to a particle in case that a likelihood is 2.05, for example, by calculating an average value (i.e., 2.05=(1.8+2.3)÷2).

The information processing apparatus 201 according to the present example embodiment is able to more accurately calculate estimation information for a target. A reason for this is that executing a fitting process can achieves a process which excludes a case where a particle has a high likelihood but has unrealistic state information.

In the present example embodiment, a scenario is generated based on a random seed, and a fitting process is executed based on state information in a generated scenario and a likelihood relating to the scenario. However, as described above, in a fitting process, the information processing apparatus 201 may execute, for example, a process of adjusting a number of random seeds depending on a scattering status of the state information and the likelihood. When a relevance calculated by the relevance determination unit 210 does not sufficiently represent a relevance between the state information and the likelihood, the assimilation calculation unit 205 further reads a random seed. Thereafter, the process illustrated in the steps S202 to S205 in Fig. 5 is executed. The above-described process provides an advantageous effect of being able to more precisely calculate estimation information relating to a target with a small number of random seeds.

Furthermore, although a fitting process is executed based on a representative evaluation value and representative state information in the above-described process, the fitting process may be executed based on all evaluation values and all pieces of state information. Moreover, as a procedure of selecting a representative value, a particle having a highest evaluation value may be selected.

A prediction unit (the prediction unit 106 in Fig. 1, or the prediction unit 206 in Fig. 4) generates state information at a next timing in each of the above-described example embodiments, but does not necessarily need to generate state information at a next timing and may generate state information at a different timing. In other words, a timing being a target for calculation by the prediction unit is not limited to the above-described example.

Next, an advantageous effect of the information processing apparatus 201 according to the second example embodiment of the present invention will described.

The information processing apparatus 201 according to the present example embodiment is able to provide information serving as a basis for accurately estimating a state relating to a target. A reason for this is similar to the reason described in the first example embodiment.

Furthermore, the information processing apparatus 201 according to the present example embodiment is able to provide, in a short time, information serving as a basis for accurately estimating a state relating to a target. A reason for this is that an assimilation process is executed in parallel.

Still further, the information processing apparatus 201 according to the present example embodiment is able to stably provide information serving as a basis for accurately estimating a state of a target. A reason for this is that estimation information having a small variation can be generated by generating estimation information based on representative state information and a representative likelihood.

<Third example embodiment>

Next, a third example embodiment of the present invention will be described.

A configuration of an information processing apparatus 301 according to the third example embodiment of the present invention will be described in detail with reference to Fig. 6. Fig. 6 is a block diagram illustrating a configuration of the information processing apparatus 301 according to the third example embodiment of the present invention.

The information processing apparatus 301 according to the third example embodiment includes a relevance determination unit (relevance determiner) 302 and an evaluation processing unit (evaluation processor) 303.

The information processing apparatus 301 is communicably connected to an estimation apparatus (simulation apparatus) 304. The estimation apparatus 304 estimates a plurality of scenarios representing an aspect of change of a target state. The estimation apparatus 304 estimates the plurality of states (or probabilistically estimates the states) in accordance with an assimilation process as described with reference to the steps S101 to S105 (Fig. 2). The information processing apparatus 301 can output, to the information processing apparatus 301, a likelihood of the scenario for observation information observed for the target, and the scenario. The information processing apparatus 301 can be implemented, for example, by using the function of the simulation unit 102 in Fig. 1 or the function of the simulation unit 202 in Fig. 4.

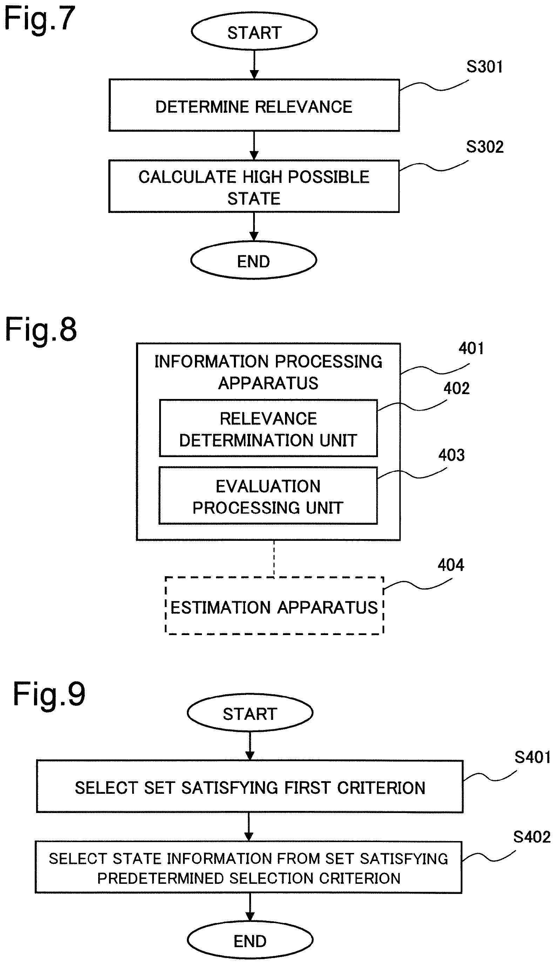

Next, a process of the information processing apparatus 301 according to the third example embodiment of the present invention will be described in detail with reference to Fig. 7. Fig. 7 is a flowchart illustrating flow of a process in the information processing apparatus 301 according to the third example embodiment.

The information processing apparatus 301 inputs a scenario estimated by the estimation apparatus 304 in relation to a target, and observation information observed in relation to the target.

The relevance determination unit 302 determines a relevance between a state (i.e., a state estimated for the target) in the input scenario and a possibility of occurrence of the state (step S301). In a process of determining a relevance, for example, the relevance determination unit 302 inputs a relevance including a parameter and determines the parameter suitable to a state in a scenario and a possibility of occurrence of the state. A process of determining a relevance is a fitting process as described above with reference to Fig. 3.

The evaluation processing unit 303 acquires a state in case that the possibility is a great value in the relevance determined by the relevance determination unit 302. In other words, based on the relevance determined by the relevance determination unit 302, the evaluation processing unit 303 acquires a predetermined selection criterion representing a criterion for selecting a high possible state (step S302). In a process illustrated in the step S302, the evaluation processing unit 303 acquires, for example, a state in case that a possibility is highest (or substantially highest) in the relevance. In this case, the evaluation processing unit 303 estimates a highest possible state in the relevance.

The relevance determination unit 302 can be implemented by using the function of the relevance determination unit 109 in Fig. 1 or the function of the relevance determination unit 210 in Fig. 4. The evaluation processing unit 303 can be implemented by using the function of the evaluation processing unit 103 in Fig. 1 or the function of the evaluation processing unit 203 in Fig. 4. Therefore, the information processing apparatus 301 can be implemented by using the function of the information processing apparatus 101 in Fig. 1 or the function of the information processing apparatus 201 in Fig. 4.

Next, an advantageous effect of the information processing apparatus 301 according to the third example embodiment of the present invention will be described.

The information processing apparatus 301 according to the third example embodiment is able to provide information serving as a basis for accurately estimating a state relating to a target. A reason for this is similar to the reason described in the first example embodiment.

<Fourth example embodiment>

Next, a fourth example embodiment of the present invention will be described.

A configuration of an information processing apparatus 401 according to the fourth example embodiment of the present invention will be described in detail with reference to Fig. 8. Fig. 8 is a block diagram illustrating a configuration of the information processing apparatus 401 according to the fourth example embodiment of the present invention.

The information processing apparatus 401 according to the fourth example embodiment includes a relevance determination unit (relevance determiner) 402 and an evaluation processing unit (evaluation processor) 403.

The information processing apparatus 401 is communicably connected to an estimation apparatus (simulation apparatus) 404. The estimation apparatus 404 estimates a plurality of scenarios representing an aspect of change of a state (state information) for a target. The estimation apparatus 404 estimates the plurality of scenarios (or probabilistically estimates the scenarios) in accordance with an assimilation process as described with reference to the steps S101 to S105 (Fig. 2). The estimation apparatus 404 can output, to the information processing apparatus 401, a possibility (a likelihood) of the scenario for observation information observed in relation to the target and the scenario. The information processing apparatus 401 can be implemented, for example, by using the function of the simulation unit 102 in Fig. 1 or the function of the simulation unit 202 in Fig. 4.

Next, a process in the information processing apparatus 401 according to the fourth example embodiment of the present invention will be described in detail with reference to Fig. 9. Fig. 9 is a flowchart illustrating flow of a process in the information processing apparatus 401 according to the fourth example embodiment.

The information processing apparatus 401 inputs a set constructed by combining a likelihood of a scenario estimated by the estimation apparatus 404 for a target and state information representing a state of the target in the scenario. In this case, the information processing apparatus 401 inputs a plurality of sets relating to a plurality of scenarios estimated by the estimation apparatus 404.

The relevance determination unit 402 selects a set satisfying a first criterion of being not far from another set or being similar to the another set in a distribution of at least a part of sets among a plurality of input sets (step S401). A distance represents a mathematical distance.

An example of a process of selecting a set satisfying the first criterion will be described.

For example, when calculating a center of all input sets, the relevance determination unit 402 calculates an average position as described with reference to Fig. 3 (one example of a remarkable position, as described with reference to Fig. 3). When calculating a center of a part of sets, the relevance determination unit 402 may calculate a center, for example, by executing a process similar to the process executed by the relevance determination unit 109. In this case, the relevance determination unit 402 selects a set including state information satisfying a predetermined constraint condition among a plurality of input sets and calculates a center of the selected set. In this case, a center to be calculated represents a mathematical center.

The relevance determination unit 402 selects a set close to the calculated center. For example, the relevance determination unit 402 determines whether or not a set is "3×σ" (a represents a standard deviation) or more away from a center and selects a set within "3×σ" from the center. Therefore, the relevance determination unit 402 selects a set satisfying the first criterion of being not far from another set or being similar to the another set.

Alternatively, as described above as a process of suppressing an influence of an outlier, the relevance determination unit 402 may select a set satisfying the first criterion by generating a cluster in accordance with a clustering method and selecting a cluster with having a large number of elements in the cluster. Therefore, a process of selecting a set satisfying the first criterion is not limited to the above-described example.

The evaluation processing unit 403 acquires state information associated with a set having a possibility satisfying a predetermined selection criterion in the sets selected by the relevance determination unit 402 (step S402). For example, a predetermined selection criterion is a criterion that a possibility is a great value. In this case, the relevance determination unit 402 acquires, for example, state information in case that the possibility is a great value in a set where the possibility is a great value. In other words, the evaluation processing unit 403 acquires a high possible state in the set selected by the relevance determination unit 402. In a process indicated in the step S402, the evaluation processing unit 403 may acquire state information associated with the possibility, for example, in a set where a possibility is highest among the sets selected by the relevance determination unit 402. In this case, the evaluation processing unit 403 acquires a highest possible state in the set selected by the relevance determination unit 402.

The relevance determination unit 402 can be implemented by using the function of the relevance determination unit 109 in Fig. 1 or the function of the relevance determination unit 210 in Fig. 4. The evaluation processing unit 403 can be implemented by using the function of the evaluation processing unit 103 in Fig. 1 or the function of the evaluation processing unit 203 in Fig. 4. Therefore, the information processing apparatus 401 can be implemented by using the function of the information processing apparatus 101 in Fig. 1 or the function of the information processing apparatus 201 in Fig. 4.

Next, an advantageous effect of the information processing apparatus 401 according to the fourth example embodiment of the present invention will be described.

The information processing apparatus 401 according to the fourth example embodiment is able to provide information serving as a basis for accurately estimating a state of a target. A reason for this is that, among a plurality of sets constructed by combining possibilities of a plurality of scenarios and state information representing a state relating to a target in the scenarios, a set having a high possibility of occurring is selected based on a center of the plurality of sets. Since the information processing apparatus 401 is able to remove, among a plurality of sets, a set lying out of a distribution relating to the plurality of sets, the information processing apparatus 401 is able to provide information serving as a basis for accurately estimating a state relating to a target.

(Hardware Configuration Example)

A configuration example of hardware resources that achieve an information processing apparatus according to each example embodiment of the present invention will be described. However, the information processing apparatus may be achieved using physically or functionally at least two calculation processing apparatuses. Further, the information processing apparatus may be achieved as a dedicated apparatus.

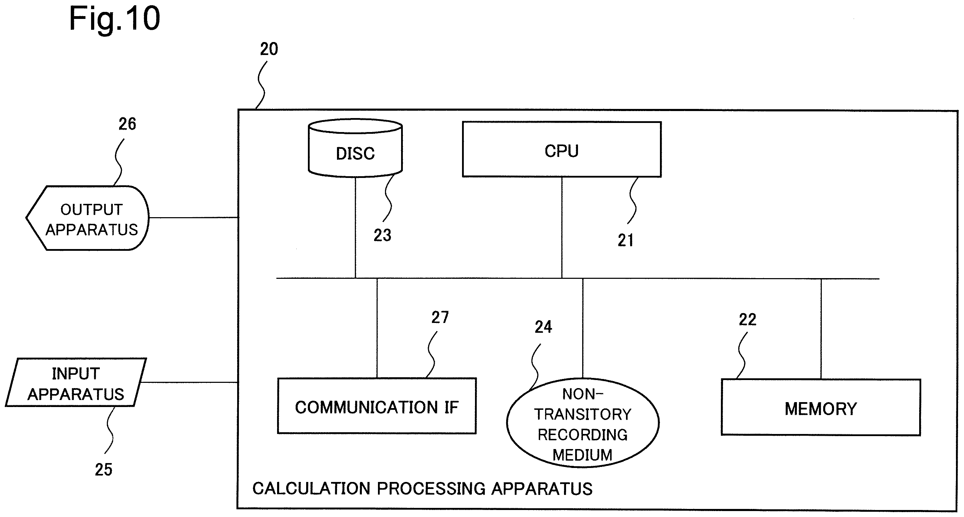

Fig. 10 is a block diagram schematically illustrating a hardware configuration of a calculation processing apparatus capable of achieving information processing apparatus according to the first to fifth example embodiments of the present invention. A calculation processing apparatus 20 includes a central processing unit (CPU) 21, a memory 22, a disk 23, a non-transitory recording medium 24, and a communication interface (hereinafter, referred to as "communication IF) 27. The calculation processing apparatus 20 may connect an input apparatus 25 and an output apparatus 26. The calculation processing apparatus 20 can execute transmission/reception of information to/from another calculation processing apparatus and a communication apparatus via the communication I/F 27.

The non-transitory recording medium 24 is, for example, a computer-readable Compact Disc, Digital Versatile Disc. The non-transitory recording medium 24 may be Universal Serial Bus (USB) memory, Solid State Drive or the like. The non-transitory recording medium 24 allows a related program to be holdable and portable without power supply. The non-transitory recording medium 24 is not limited to the above-described media. Further, a related program can be carried via a communication network by way of the communication I/F 27 instead of the non-transitory recording medium 24.

In other words, the CPU 21 copies, on the memory 22, a software program (a computer program: hereinafter, referred to simply as a "program") stored in the disk 23 when executing the program and executes arithmetic processing. The CPU 21 reads data necessary for program execution from the memory 22. When display is needed, the CPU 21 displays an output result on the output apparatus 26. When a program is input from the outside, the CPU 21 reads the program from the input apparatus 25. The CPU 21 interprets and executes an information processing program (Fig. 2, Fig. 5, Fig. 7 or Fig. 9) present on the memory 22 corresponding to a function (processing) indicated by each unit illustrated in Fig. 1, Fig. 4, Fig. 6, or Fig. 8 described above. The CPU 21 sequentially executes the processing described in each example embodiment of the present invention.

In other words, in such a case, it is conceivable that the present invention can also be made using the information processing program. Further, it is conceivable that the present invention can also be made using a computer-readable, non-transitory recording medium storing the information processing program.

The present invention has been described using the above-described example embodiments as example cases. However, the present invention is not limited to the above-described example embodiments. In other words, the present invention is applicable with various aspects that can be understood by those skilled in the art without departing from the scope of the present invention.

This application is based upon and claims the benefit of priority from Japanese patent application No. 2017-010441, filed on January 24, 2017, the disclosure of which is incorporated herein in its entirety.

[Reference signs List]

| | |
|---|---|
| 101 | information processing apparatus |
| 102 | simulation unit |
| 103 | evaluation processing unit |
| 104 | data obtainment unit |
| 105 | assimilation calculation unit |
| 106 | prediction unit |
| 107 | calculation stop determination unit |
| 108 | evaluation value calculation unit |
| 109 | relevance determination unit |
| 110 | state calculation unit |
| 151 | observation apparatus |
| 152 | input apparatus |
| 153 | output apparatus |
| 154 | observation information storage unit |
| 155 | setting information storage unit |
| 201 | information processing apparatus |
| 202 | simulation unit |
| 203 | evaluation processing unit |
| 204 | data obtainment unit |
| 205 | assimilation calculation unit |

206     prediction unit
207     calculation stop determination unit
208     statistic processing unit
209     evaluation value calculation unit
210     relevance determination unit
211     state calculation unit
212     random seed information
301     information processing apparatus
302     relevance determination unit
303     evaluation processing unit
304     estimation apparatus
401     information processing apparatus
402     relevance determination unit
403     evaluation processing unit
404     estimation apparatus
20      calculation processing apparatus
21      CPU
22      memory
23      disk
24      non-transitory recording medium
25      input apparatus
26      output apparatus
27      communication IF
501     observation information
502     observation model
503     system model
504     state estimation

**Claims**

1.  The information processing apparatus comprising:

    relevance determination means for determining relevance between status information representing a state of a target and a possibility of a scenario representing aspect of change of the state information; and
    evaluation processing means for determining state information in case that the possibility satisfies a predetermined selection criterion based on the relevance determined by the relevance determination means.

2.  The information processing apparatus according to claim 1, wherein
    the evaluation means calculates the state information with having maximum or substantially maximum probability.

3.  The information processing apparatus according to claim 1 or claim 2 further comprising:

    calculation means for calculating a possibility during a period included in the scenario based on the possibility, wherein
    the scenario includes possibilities at a plurality of timings.

4.  The information processing apparatus according to any one of claims 1 to 3, wherein
    the relevance determination means determines the relevance by using a set in which the state satisfies a predetermined criterion among sets that includes the state information included in the scenario and a possibility of the scenario.

5.  The information processing apparatus according to any one of claims 1 to 3, wherein
    the relevance determination means classifies sets that includes the state information included in the scenario and a possibility of the scenario into a plurality of cluster, and determines the relevance by using a cluster with having large number of the sets among the classified plurality of clusters.

6.  The information processing apparatus according to any one of claims 1 to 5, wherein
    the relevance represents a convex function or a Gaussian function.

7. The information processing apparatus according to claim 3, wherein
processing means; wherein
the calculation means calculates a possibility during the period by average possibility of the plurality of the scenarios,
the processing means calculates first weighted average of the state information by using, as weight, the calculated possibility during the period and calculates second weighted average of the average possibility by using, as weight, the calculated possibility during the period, and
the relevance determination means determines the relevance based on the first weighted average and the second weighted average calculated by the processing means.

8. The information processing apparatus according to claim3, wherein
processing means; wherein
the calculation means calculates a possibility during the period by average possibility of a plurality of the scenarios,
the processing means selects state information and average possibility with having a large possibility during the period, and
the relevance determination means determines the relevance based on the state information selected by the processing means and the average possibility selected by the processing means.

9. An information processing method, by a calculation processing apparatus, comprising:

   determining relevance between status information representing a state of a target and a possibility of a scenario representing aspect of change of the state information; and
   determining state information in case that the possibility satisfies a predetermined selection criterion based on the determined relevance.

10. A recording medium storing an information processing program causing a computer to achieve:

   a relevance determination function for determining relevance between status information representing a state of a target and a possibility of a scenario representing aspect of change of the state information; and
   an evaluation processing function for determining state information in case that the possibility satisfies a predetermined selection criterion based on the relevance determined by the relevance determination function.

Fig.1

## Fig.2

```
                              ┌─────────┐
                              │  START  │
                              └─────────┘
                                   │
                                   ▼
                           ◇─────────────◇        S102
                          ╱  IS THERE    ╲
                         ╱  OBSERVATION   ╲───── NO
                         ╲  INFORMATION?  ╱
                          ╲              ╱
                           ◇───────────◇
                                │ YES
                                ▼
                        ┌──────────────────┐      S103
                        │ EXECUTE ASSIMILATION │
                        │      PROCESS      │
                        └──────────────────┘
                                │
                                ▼
   ┌──────────────┐     ┌──────────────────┐      S104
   │ NEXT TIMING  │     │ GENERATE STATE   │◄─────
   └──────────────┘     │  INFORMATION     │
      S101              └──────────────────┘
                                │
                                ▼
                           ◇─────────────◇        S105
                          ╱ IS ASSIMILATION╲
                  NO ────╱ PROCESS STOPPED? ╲
                         ╲                  ╱
                          ◇───────────────◇
                                │ YES
                                ▼
                        ┌──────────────────┐      S106
                        │ CALCULATE EVALUATION │
                        │      VALUE       │
                        └──────────────────┘
                                │
                                ▼
                        ┌──────────────────┐      S107
                        │ CALCULATE RELEVANCE │
                        └──────────────────┘
                                │
                                ▼
                        ┌──────────────────┐      S108
                        │ GENERATE STATE   │
                        │  INFORMATION     │
                        └──────────────────┘
                                │
                                ▼
                            ┌─────────┐
                            │   END   │
                            └─────────┘
```

# Fig.3

EVALUATION VALUE

STATE VALUE

# Fig.4

**INFORMATION PROCESSING APPARATUS** — 201

**SIMULATION UNIT** — 202
- DATA OBTAINMENT UNIT — 204
- ASSIMILATION CALCULATION UNIT — 205
- PREDICTION UNIT — 206
- CALCULATION STOP DETERMINATION UNIT — 207
- STATISTIC PROCESSING UNIT — 208

**EVALUATION PROCESSING UNIT** — 203
- EVALUATION VALUE CALCULATION UNIT — 209
- RELEVANCE DETERMINATION UNIT — 210
- STATE CALCULATION UNIT — 211

SEED INFORMATION — 212

OBSERVATION INFORMATION STORAGE UNIT — 154
SETTING INFORMATION STORAGE UNIT — 155

OBSERVATION APPARATUS — 151
INPUT APPARATUS — 152
OUTPUT APPARATUS — 153

EP 3 575 992 A1

# Fig.5

```
         START
           │
           ▼
┌─────────────────────┐        S201
│  ALLOCATE RANDOM SEED│
│     INFORMATION      │
└─────────────────────┘
           │
           ▼
┌─┬─────────────────┬─┐        S202
│ │ EXECUTE ITERATION │ │
└─┴─────────────────┴─┘
           │
           ▼
┌─────────────────────┐        S203
│ EXECUTE STATISTIC    │
│ PROCESSING OF RESULT │
└─────────────────────┘
           │
           ▼
┌─────────────────────┐        S204
│ CALCULATE EVALUATION │
│        VALUE         │
└─────────────────────┘
           │
           ▼
┌─────────────────────┐        S205
│    EXECUTE FITTING   │
└─────────────────────┘
           │
           ▼
┌─────────────────────┐        S206
│ GENERATE ESTIMATION  │
│     INFORMATION      │
└─────────────────────┘
           │
           ▼
          END
```

# Fig.6

```
┌────────────────────────────────┐  301
│   INFORMATION PROCESSING        │
│        APPARATUS                │
│  ┌──────────────────────────┐   │  302
│  │  RELEVANCE DETERMINATION │   │
│  │          UNIT            │   │
│  └──────────────────────────┘   │  303
│  ┌──────────────────────────┐   │
│  │  EVALUATION PROCESSING   │   │
│  │          UNIT            │   │
│  └──────────────────────────┘   │
└────────────────────────────────┘
                │
    ┌ ─ ─ ─ ─ ─ ─ ─ ─ ─ ─ ─ ┐       304
      ESTIMATION APPARATUS
    └ ─ ─ ─ ─ ─ ─ ─ ─ ─ ─ ─ ┘
```

# Fig.7

```
        ( START )
            │
            ▼
┌───────────────────────┐         S301
│  DETERMINE RELEVANCE  │
└───────────────────────┘
            │
            ▼
┌───────────────────────┐         S302
│ CALCULATE HIGH POSSIBLE│
│        STATE          │
└───────────────────────┘
            │
            ▼
         ( END )
```

# Fig.8

```
┌───────────────────────────────────┐     401
│  INFORMATION PROCESSING            │
│       APPARATUS                    │     402
│  ┌─────────────────────────────┐   │
│  │       RELEVANCE             │   │
│  │   DETERMINATION UNIT        │   │     403
│  └─────────────────────────────┘   │
│  ┌─────────────────────────────┐   │
│  │       EVALUATION            │   │
│  │    PROCESSING UNIT          │   │
│  └─────────────────────────────┘   │
└───────────────────────────────────┘
              ┆
┌ ─ ─ ─ ─ ─ ─ ─ ─ ─ ─ ─ ─ ─ ─ ─ ┐     404
│   ESTIMATION APPARATUS          │
└ ─ ─ ─ ─ ─ ─ ─ ─ ─ ─ ─ ─ ─ ─ ─ ┘
```

# Fig.9

```
              ( START )
                  │
                  ▼
┌───────────────────────────────────────────┐     S401
│   SELECT SET SATISFYING FIRST CRITERION     │
└───────────────────────────────────────────┘
                  │
                  ▼
┌───────────────────────────────────────────┐     S402
│ SELECT STATE INFORMATION FROM SET SATISFYING│
│   PREDETERMINED SELECTION CRITERION         │
└───────────────────────────────────────────┘
                  │
                  ▼
               ( END )
```

# Fig.10

OUTPUT APPARATUS — 26

INPUT APPARATUS — 25

20

DISC — 23

CPU — 21

COMMUNICATION IF — 27

NON-TRANSITORY RECORDING MEDIUM — 24

MEMORY — 22

CALCULATION PROCESSING APPARATUS

# Fig.11

502

| OBSERVATION INFORMATION | — | OBSERVATION MODEL |
501

| SYSTEM MODEL |
503

| STATE ESTIMATION |
504

## INTERNATIONAL SEARCH REPORT

| International application No. |
| --- |
| PCT/JP2018/001160 |

**A. CLASSIFICATION OF SUBJECT MATTER**
Int.Cl. G06F19/00(2018.01)i

According to International Patent Classification (IPC) or to both national classification and IPC

**B. FIELDS SEARCHED**

Minimum documentation searched (classification system followed by classification symbols)
Int.Cl. G06F19/00

| Documentation searched other than minimum documentation to the extent that such documents are included in the fields searched | |
| --- | --- |
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y<br>A | JP 2013-200683 A (NIPPON TELEGRAPH AND TELEPHONE CORP.) 03 October 2013, in particular, paragraph [0016] (Family: none) | 1-4, 6, 9, 10<br>5, 7, 8 |
| Y<br>A | JP 2016-71383 A (INTERNATIONAL BUSINESS MACHINES CORPORATION) 09 May 2016, in particular, paragraphs [0009], [0010], [0069] & US 2016/0092782 A1, in particular, paragraphs [0017], [0018], [0074] | 1-4, 6, 9, 10<br>5, 7, 8 |
| Y<br>A | JP 2016-4525 A (HITACHI, LTD.) 12 January 2016, in particular, claims 1, 2 (Family: none) | 4, 6<br>1-3, 5, 7-10 |
| A | WO 2014/141344 A1 (NEC CORP.) 18 September 2014, entire text, all drawings & US 2016/0042101 A1, entire text, all drawings | 1-10 |

☐ Further documents are listed in the continuation of Box C. ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 22 March 2018 (22.03.2018) | 03 April 2018 (03.04.2018) |

| Name and mailing address of the ISA/<br>Japan Patent Office<br>3-4-3, Kasumigaseki, Chiyoda-ku,<br>Tokyo 100-8915, Japan | Authorized officer |
| --- | --- |
| | Telephone No. |

Form PCT/ISA/210 (second sheet) (January 2015)

**EP 3 575 992 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5340228 B
- WO 2016031174 A
- JP 2017010441 A